(19) Europäisches Patentamt

European Patent Office

Office européen des brevets

(11) EP 0 859 236 A1

(12) EUROPEAN PATENT APPLICATION

(43) Date of publication:
19.08.1998 Bulletin 1998/34

(51) Int. Cl.⁶: G01N 33/28, G01N 21/35

(21) Application number: 97430007.1

(22) Date of filing: 14.02.1997

(84) Designated Contracting States:
FR

(71) Applicants:
• BP CHEMICALS S.N.C.
92400 Courbevoie (FR)
• BP Chemicals Limited
London EC2M 7BA (GB)

(72) Inventor:
The designation of the inventor has not yet been filed

(74) Representative:
Lassalle, Pierre-Dominique et al
BP Chemicals S.N.C.,
Sce Propriété Industrielle LPID,
BP no. 6
13117 Lavera (FR)

(54) Determination of properties of oil

(57) A method of controlling a separation of a component in crude oil e.g. downstream of a well head, in which the oil is analysed e.g. by near infra red (NIR) spectroscopy, at or before a pipeline between the analyser and separator especially a line at least 10Km long and the results used to optimise the separation. NIR spectroscopy is also used to determine a property e.g. composition of a crude oil, in particular its content of naphtha, gas oil and/or fuel oil.

FIG. 2

EP 0 859 236 A1

## Description

This invention concerns methods of determining properties of oil, especially crude oil.

Many properties of crude oil are regularly determined on a very large scale world wide, especially gas content and percentage of naphtha, gas oil and fuel oil, the latter being a key parameter in assessing the value of any oil. These determinations are usually done off line by mechanical techniques e.g. distillation.

The present invention provides a method of controlling a process for separating from a crude oil at least one component of said crude oil, which flows into a pipeline and then through a separator, wherein a sample of the crude oil before entering the pipeline is analysed for at least one component on or at line to give an analytical result before the crude oil, from which the sample was taken, reaches the separator, and the operation of the separator is adjusted based on said result to control the separation of said component. Preferably the adjustment is to optimise the separation of said component from said crude oil, especially when the component comprises compounds which are gases under the conditions of the separation, in particular at least one hydrocarbon of 1-5 carbons.

The sample of crude oil may be analysed just before its entry to the pipeline leading to the separator which pipeline may be 0.1-1000Km, especially 1-500Km or 100-400Km long before the separator or at a point intermediate in said line e.g. at an intermediate pumping station (but still preferably at least 0.1Km or 1Km from said separator). The said pipeline is usually downstream of the well head and may be substantially horizontal (in relation to its length), and may contain crude oil under pressure e.g. with gas, usually with water, being transported to the separator from a collection point fed by separate well heads or from a single well head. The pipeline prior to the separator may be underground upstream of the well head, i.e. with a significant vertical component to its length; the separator may be at the well head.

The time taken for the crude oil to pass from the point of analysis to the separator may be 0.2 hr to 10 days, especially 0.5-5 days or 1-10 hrs. The analysis may be chromatographic e.g. gas phase or high liquid phase chromatographic, in which a sample of the crude oil is injected periodically into a column on or at line. Preferably the analysis is spectroscopic e.g. UV, visible (200-800nm) infra red (e.g. 2600nm - 10000nm) but especially is near infra red e.g. as described further below. The spectroscopic analysis is in a standard cell kept at a fixed temperature through which cell the appropriate radiation passes, the path length of the cell varying with the wavelength e.g. from 0.1-1mm for IR, 0.5-10mm for NIR, 1-5cm for visible and 5-10cm for UV radiation.

The output of the analysis in the form of a percentage of an ingredient may be relayed by telephone or radio to the controller of the separator, where it can be considered in relation to the future operation of the separator, so that when the crude oil analysed reaches the separator, the operating conditions e.g. flow rate, temperature and/or pressure may be such as to optimise the separation e.g. optimise the removal of natural gas. Preferably however the output of the analysis as a percentage of component or otherwise e.g. absorption (or derivatives thereof may be transmitted electrically or by electromagnetic radiation to a computer (in relation to the separator) which can then be programmed for feed back control, e.g. knowing what the gas content of the oil will be when that oil reaches the separator, the conditions can be changed to optimise throughput of gas.

The analysis may be performed in real time or near real time.

The present invention also provides a method of determining or predicting a value of a property of a material which is a crude oil, or product of a process to which said crude oil is a feed or yield of said process, which comprises measuring the absorption of said oil at least one wavelength in the region 600-2600nm and converting that absorption (or derivative thereof) into a value of said property. The conversion may be direct, or indirect by statistical correlation or by non correlation techniques.

The application of near infra red spectroscopy to the crude oil has been found to enable the data to be obtained on line or at line and very quickly, resulting in suitability for automated control techniques.

The crude oil is usually primarily aliphatic in nature, but may contain up to 50% w/w liquid aromatic compounds. It is usually an oil field product from a well head, as whole well product i.e. the multiphase mixture from the well bore containing oil and water and/or gas which may be at 50-200 bar pressure, or one such product after at least partial removal of water and/or gas ready for sending away down a pipeline from the well head and may be at up to 50 bar e.g. 1-10 bar pressure. It may be on a production platform, or between platforms or from a production platform to a collection or storage facility, on or offshore, or vessel, or at such a collection or storage facility or downstream thereof e.g. in a pipeline downstream thereof e.g. at a further storage facility, such as in a gathering station or refinery or prior to a separation facility e.g. to separate gas and/or water and/or other components of the crude oil e.g. in a distillation to recover e.g. naphtha. The crude oil leaving the well head may contain dissolved gas (e.g. in amounts of up to 15% by weight gas e.g. 1-10% wt) and/or water or water droplets (e.g. 5-40% wt. water), while gas and/or water may be present as a physically separate phase especially when arriving at the well head; the Gas/Oil ratio (GOR) (expressed in StCu ft gas per barrel of oil) may be 1-10000 e.g. 2000-9000 (for oil arriving at a well head) and 1-1000 e.g. 50-500 or 3-50 (especially for partly degassed oil leaving a well head). The crude oil may contain dissolved gaseous hydrocarbons e.g. methane, ethane, propane and butane (each in amounts of 0.1-5% e.g. 0.5-5% by weight or 0.1-10% e.g. 0.5-10% in the case of methane) and in total up to 15% especially to 10%. The oil may also be present with other gases e.g. inert gas such as nitrogen and carbon dioxide (each in amount of 0.1-5% such as 1-5 or 0.1-1%). In relation to the total weight of liquids in solution in the crude oil, the crude oil may contain 0-40% e.g. 10-30% gas condensates or light ends (which may be preferably in the amount relative to naphtha which was present in the well head crude oil, and so the analysed crude does not contain added condensates) 0-40% e.g. 20-40% by weight of naphtha, 5.30% e.g. 10-20% kerosene, 0.50% e.g. 5-40% or 20-30% gas oil, 0-40% e.g. 5-40% or 20-40% fuel oil (with residue). It may also contain (expressed on the same basis) 0-8% asphaltenes (e.g. 0.01-2%) 0-8% e.g. 1-7% or 0.05-2% sulphur and 0-10 e.g. 1-6mg KOH/g as an expression of the acidity. The percentage composition of the total of dissolved gaseous hydrocarbons, gas condensates and light ends (expressed on the same basis) may be 5-50%, such as 10-40% especially 10-30% or 15-40%; such a combination may be that boiling at up to 130°C under atmospheric pressure. Its API gravity may be 10-60 e.g. 20-55 and its Pour Point -60°C to + 60°C e.g. -20°C to +20°C. Its boiling range may be -30°C to 550°C and final boiling point up to 880°C. The oil may have been dewatered and/or desalted before analysis and in particular when analysed may be substantially free of any separate gas phase. It may be substantially free of dissolved gas when analysed, but preferably contains 0.1-15% dissolved gas. The oil being sampled may contain up to 10% wt suspended solids e.g. 1-5%, but is preferably substantially free of suspended solids.

The oil is preferably substantially free of suspended organic solids, such as insoluble bitumen and tar sands and asphaltenes, and suspended inorganic solids such as formation rock.

The wavelength(s) at which the absorption is measured is/are in the range 600-2600 nm, e.g. 600-1000, 1000-1500, 1500-2000 or 2000-2600 nm, and may represent the first second, or third overtone region or the combination region for CH bonds. The NIR analysis is preferably primarily by transmittance especially substantially completely by transmittance; reflectance analysis may be used. The oil may be analysed as a liquid sample in the cell of an NIR spectrometer or in a remote cell fitted with optical fibre guides leading to and from such a spectrometer, there being one or more cell per spectrometer, e.g. with the optical fibres passing through a multiplexer, so one spectrometer can analyse successively the liquids in a series of cells. When the oil is in the presence of gas, either dissolved or separate, so it is under pressure, the cell itself is pressurised. The cell may receive a separate liquid sample e.g in a laboratory separate from the source of the sample or at line next to the source or may preferably be in line, e.g. in a side line off main line containing the material to be tested. Because the oil often contains suspended solids, the cell may become fouled periodically and fouling should be removed or compensation made for it (e.g. by comparison of the absorption of the cell when empty but containing fouling and when containing analysate). Preferably insolubles in the crude oil sampled are substantially removed before the NIR analysis.

The property to be determined may be chemical, physical, physicochemical or optical or mechanical. Examples of such properties are composition e.g. percentage of one or more components e.g. soluble hydrocarbons, especially such as gaseous hydrocarbons, in total or each e.g. methane, ethane, propane, isobutane or butaneor mixtures thereof e.g. lean gas, natural gas, butanes and condensates, also, sulphur compounds, acid compounds, asphaltenes, wax, aromatics. Percentage compositions of the total gaseous hydrocarbons, with gas condensates may be obtained as such or in combination also with light ends e.g. $C_{6-8}$ alkanes in the form of the percentage of components distilling at up to 130°C (under atmospheric pressure).

Among physical properties are viscosity, specific gravity (or API gravity), wax point, total boiling range, Total boiling point (TBN), Total acidic number (TAN). The property determined may be a mixture of properties e.g. gas/oil ratio, which relates to the ratio of vapour phase to total liquid phase (i.e. including water if present and dissolved gas if present). Other properties of the oil/or yields of products from the oil include percentages of one or more of gasoline, naphtha, gas oil, fuel oil and residue.

Once the absorption on the oil has been made, the absorption value is converted into a value for the desired property. Where the wavelength(s) of the absorption is/are characteristic of the compound to be analysed this conversion may be direct, for example by absorption e.g. in the mid infra red region, where methane is known to absorb, the percentage of methane can be determined directly according to the Beer Lambert law; usually the cell is calibrated with appropriate media containing known concentrations of methane, or the absorbance is compared directly to another parallel cell containing that medium.

Preferably however the wavelength(s) chosen for the absorption are chosen by statistical means. These are correlative and involve relations of a regressional character between the property(ies) and the absorbances. These Multivaiable analyses include multilinear regression (MLR), Principle Component Regression (PCR), Canonic regression and regression by Partial Least Squares (PLS). The relation between property and absorbance the Regression Equation may be linear e.g. Property value = $\Sigma$ (Di times factor i) where where Di is the absorption, which may optionally be normalized, or first, second or third derivative thereof at the specific wavelength and factor i is the Regression Coefficient i.e. the value of the significance of the absorption at that wavelength to the property, the sum being taken over all the wavelengths whose absorptions are considered. The relation is more usually quadratic or involves fractions or is of higher algebraic form. In each case the Regression Equation has to be determined empirically, using a calibration set with known properties. This approach is useful when the calibration set encompasses the samples being tested, but, when e.g. a crude oil from a new source outside the previous calibration set is analysed, the Regression Equations will need redetermination to take the new oil into account. Many commercial spectrometers have integrated computerised software to do this statistical analysis and generate the Regression Equation. Examples of these approaches applied to other oils are described in EP-A-285251, 304232 and 305090, the disclosure of which is hereby incorporated by reference.

Examples of suitable correlation techniques as applied to crude oil are use of PLS on crude oils to determine the optimum wavelengths for determination of their gas content. Suitable wavelengths include 2173, 2295, 2365, 2406, 2439 and 2500nm, especially all of these, especially in a regression equation for normalized absorbances in the form.

Wt % gas in crude oil = - [A x Abs1]-[B x Abs2]-[C x Abs3]-[D x Abs4]+[E x Abs5] - [F x Abs6] + G

where Abs 1-6 are the normalised absorbances at the 1-6 wavelengths 2173-2500nm specified above and A-G are numbers, in particular A and B each being 14000-15000 e.g. 14450-14550, C being 19300-20300 in particular 19800-19900, D being 14800-15800, in particular 15320 to 15420, E being 6000-7000, e.g. 6520 to 6620, F being 8600-9600 e.g. 9070-9170 and G being 460-470 in particular 465-468.

A more preferred method involves measurement of absorption at more than one wavelength and then, without any statistical treatment, comparing the absorption (or a derivative thereof) of the unknown with the absorptions (or derivative) of a number of standard crude oils of known properties in a bank to determine the standard(s) which have the nearest absorption values at the same wavelengths to the unknown; the property of the unknown is then the same as that of the chosen standard (or the averaged value of the chosen standards). This technique is described further in WO 96/11399, the disclosure of which is hereby incorporated by reference. The method as applied to crude oil comprises determining or predicting a value $P_x$ which is a value of a property of a material X, which is a crude oil, or a property of a product of a process from said material or yield of said process, which method comprises measuring the absorption $D_{ix}$ of said material at more than one wavelength in the region 600-2600 nm, comparing said signals indicative of said absorptions or a mathematical function thereof with signals indicative of absorptions $D_i m$ at the same wavelengths or a mathematical function thereof for a number of standards S in a bank for which the said property or yield P is known, and choosing from the bank at least one standard $S_m$ with property $P_m$ said standard having the smallest average value of the absolute difference at each wavelength i between the signal for the material and the signal for the standard $S_m$ to obtain $P_x$, with averaging of said properties or yields $P_m$ when more than one standard $S_m$ is chosen. Preferably, the standard $S_m$ chosen for the property or yield wanted is such that in relation to the unknown X and each chosen standard $S_m$ the following functions are met,

when $i_{xm} < 1_{min}$ then $P_x - P_m \leq$ experimental error in P

where $P_x$ is property of unknown X, $P_m$ is property of chosen standard $S_m$, $i_{xm}$ is defined by $i^2 (xm) = \Sigma (D_{ix} - D_{im})^2$ and the $i_{min}$ is defined by the proximity index, which is the minimum value in relation to 2 standards Sa and Sb with properties $P_a$ and $P_b$ for which $P_a - P_b < E\sqrt{2}$, where E is the experimental error in determining said property or yield in the standard.

The proximity index is usually less than the minimal index $i_m$ which has been determined from the standards Sa, Sb, Sc.... by (a) calculating for each pair of standards Sa/Sb, Sa/Sc the value of $i^2(a,b)$ etc, (b) relating the values of $i^2(a,b)$ etc to the corresponding differences EP (ab) in properties Pa, Pb etc (c) calculating for each value L, for which i (ab) is L, the average of the corresponding differences Epab, (d) determining Minimal index from the value of minimal index i (ab) where 95% of Epab is the same as reproducibility standard for the property.

The crude oil may be tested on line, at line or off line. The use of NIR according to this invention is quick and much cheaper than the off line long time consuming analytical techniques involving distillation hitherto used; the latter were not suitable for on line control of operations, while those of the invention are.

The crude oil when analysed may be free of dissolved gas but preferably contains dissolved gas e.g. at 5-20 bar pressure and may also contain a discrete gaseous phase comprising the gas. This crude oil may be analysed by the NIR spectrometer and the property of the oil determined directly. The crude oil analysed may be a feed to a separating means e.g. a gas separator to separate gas from liquid phase (either oil or water or both) or a distillation to separate gas and one or more liquid phases, e.g. naphtha, gas oil and/or fuel oil.

One use of this approach is to value the oil, which is determined from its naphtha, gas oil and fuel oil contents. Thus in this approach a crude oil is analysed by NIR for these components and, from the percentages of each, and the known value of each of the 3 isolated components, the value for that batch of crude oil can be determined. This is useful directly for commercial purposes as such, but also especially in relation to operations of pipelines containing oil from wells with different owners, as described further below.

The analysis by NIR may be before a separation step in which one or more components may be separated from the rest e.g. gas in total or separately such as dry gas (predominantly methane and ethane) propane or butane. The separation step may be to remove substantially all the gas at once, e.g. lowering the pressure to a millibar level e.g. 1-200 or 10-150 millibar level at 30-50°C, but preferably the gas is removed in stages by stepwise reduction in pressure e.g. from 10-20 bar at 40-60°C, to 1-3 bar at 35-50°C to 10-150m bar at 30-40°C. In some cases there is a critical size to the separation apparatus, or a critical maximum throughput of one of the components; for example in gas separators especially cyclones, there is an maximum throughput of gas through an apparatus of a particular size. Using the separator with less than the optimum throughput is wasteful. NIR analysis is applied to the feed mixture of crude oil and gas and/or water to determine its content of the critical component. Then the flow rate of the mixture is adjusted to optimise the total weight or value of the critical component through the separator and by continuous or continual analysis e.g. by on line analysis, the flow rate is continuously or continually adjusted. In this way there are significant technical benefits in the separation step. These benefits are found even more when there is a length of pipeline between the point of analysis and the separating means e.g. at least 1 Km, especially at least 10 Km such as 1-1000 or 5-50 or 50-500 Km. In this embodiment, the oil is analysed by NIR before entering the pipeline, or in transit in the pipeline e.g. at an intermediate point such as a pumping station, and its flow rate adjusted in response to its content of the critical component; the pipeline then carries the oil with the optimum flow rate of critical component to the separation means. By controlling the flow in this way the use of the separation means is optimised. The content of the critical component often varies with time, so, without adjustment of the flow rate of that component, the separator may not be operating optimally, however the flow rate should be adjusted according to the changes in the critical component content within the capacity of the pipeline. Hence if the gas content reduced suddenly, then the flow rate should be gradually increased. Instead of controlling the flow rate of the oil and hence optimizing the flow rate of the critical component, the information on the content of that component and its instantaneous flow rate can be passed to the operations of the separation means, so that it can be adjusted to be optimum for that particular oil/component mix when it reaches the separator e.g. by changing the temperature pressure or flow rates. Instead of controlling the separator based on the gas content, it may be controlled based on a property of a degassed crude oil e.g. TBP.

The variation in gas or other component content is often significant when the pipeline is fed from a collection station into which pass oils from more than one well, e.g. when one or more wells is suddenly brought on or off stream. In this case the analysis may still be on the oil just before entry to the pipeline e.g. the blend of oils for example at a collection station offshore or onshore but preferably the individual oils are analysed before blending either at the collection station itself (e.g. with one spectrometer per line or with multiplexer devices and one spectrometer per blend), or at the entry of each oil into its subsidiary pipeline leading to the collection station e.g. at each separate well head e.g. platform or seabed, optionally either before or preferably after any separation of water. Analysis at the entry of each oil into its subsidiary pipeline is particularly useful when the collection station has at least one branched pipeline feeding it. Analysis of the oil from each well head separately provides the operation of changing flow rate with maximum lead time based on the residence time of the oil from that well head in the subsidiary pipelines. This approach is optimised further according to a further embodiment of the invention in which NIR absorption at one or more wavelengths is measured on each feed to a blend in order to predict one or more properties of the blend. By this means with a number of platforms with equal residence time of flow to a central collection station, analysis of the oil on each platform at one instant give an analysis of the blend formed at the collection station a time equal to that residence time later; when the residence times are different, the NIR analysis of the oil leaving each platform in any line at an appropriate lead time equal to the residence time in the line can be combined to give the analysis of the blend which will result from mixing of these oils. While the NIR absorbances on each platform can be converted to properties and those in turn used to predict the analysis of the later blend, advantageously the absorbances are directly converted into properties of the blend. This is achieved via the statistical or nonstatistical approach described above, adapted as required to cope with different residence times for the oils in the subsidiary pipelines.

A further application for NIR analysis exists in relation to the blending of a number of oils from different well heads. Information on the value of the part of the oil in a blend derived from that well head oil is often useful both for internal accounting (e.g. to see which wells contribute compositionally most to the blend) and for external accounting in relation to distribution of value of the blended oil to each well head owner. This approach in relation to the values of the blend and its apportionment applies whether the blend is sold as such, passed down a pipeline e.g. onshore to a gathering station or then degassed in a gas separator.

In a further embodiment in relation to passage of oil down a pipeline, NIR analysis can be applied in relation to identification of individual oils leaving the line. Thus the analysis can be applied to the oil before entry and the oil analysed continuously on exit and monitored to identify the exit of any particular fraction. This may be done by measuring at least some NIR absorbances on entry and at least some on exit and using the statistical or non-statistical approach described above to determine which oil fraction leaving the line is nearest that of the entry oil.

The invention and its method of operation are illustrated in and with respect to the accompanying drawings in which:

Fig. 1 and Fig. 4 are schematic diagrams of blending process operations of the invention.
Fig. 2 is a schematic diagram of a transport and separation process operation.
Fig. 3 is a schematic diagram of a blending transport and separation process operation.

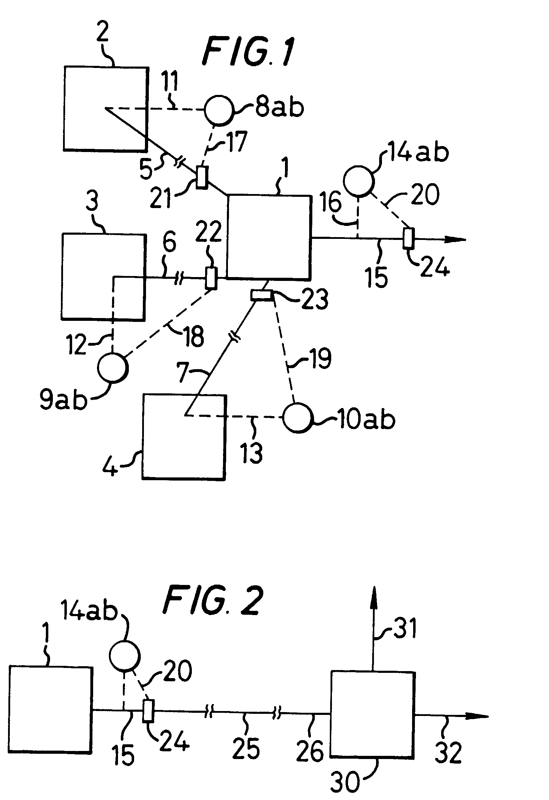

Referring to Fig. 1 a collection station 1 and three well head locations 2, 3, 4 e.g. platforms offshore are joined to station 1 by pipelines e.g. subsea pipelines 5, 6 and 7 respectively. Analytical equipment 8-10 comprising spectrometers 8a-10a and computers 8b-10b are joined to line 5, 6 and 7 by analytical lines 11-13 respectively, while analytical equipment 14 comprising spectrometer 14a and computer 14b is attached by analytical line 16 to a line 15 leaving station 1; the analytical lines 11-13 and 16 may be optical fibres and are joined to the appropriate line or to a sample line therefrom. Signal transmission means 17-20 connect computers 8a-10a and 14a to pumps 21-24; the means 17-20 may be wire/cable or electronic means.

In use the absorption at the appropriate wavelengths in the NIR region of the oil feed in location 2 is measured by means of spectrometer 8a and the absorbances interpreted by computer 8b and converted into analytical results e.g. % naphta, gas oil and fuel oil. The same operations are performed in locations 3 and 4. From the analytical results and the feed rates, the value of the oil leaving each location can be determined. In this operation, the signal transmission means 17-20 to the pumps are not used.

In a further operation the computers 8b-10b pass signals to the corresponding pumps 21-23 which instruct them to adjust their flow rates for material in lines 5-7 according to a preset plan e.g. to keep the analysis of the blend leaving in line 15 for at least one component e.g. the gas content in a preset range e.g. ± 10% or ± 5% of a specified value.

Referring now to Fig.2, a station 1, which may be a collection station as in Fig. 1 (for combining more than one oil) or may be supplied with oil from one well head, has a line 15 leading to pump 24, with analytical equipment 14ab, joined to line 15 by fibre 16 and to pump 24 by signal transmission means 20, which may be wire/cable or electronic. Line 15 leads to pipeline 25, which extends e.g. under the sea to a line 26 to separating means 30, separating means 30 has line 31 for separation of one fraction e.g. gas and line 32 for separation of another fraction e.g. degassed crude oil for further transport e.g. by pipeline or ship or passage to a refinery.

In use, the oil containing a specified component e.g. gas leaving station 1 in line 15 is analysed by NIR for said component by equipment 14a and the result combined with the flow rate of liquid in line 15 to provide the total flow rate of that component in line 15 at that time. In one embodiment of the invention, that flow rate information is passed to the operation of the separating means 30 so that the separator of that component in line 31 can be optimized, when the oil in that line reaches the separating means e.g. by changing the separation conditions of temperature or pressure; this latter can be checked by means not shown by analysis of the liquid entering separating means 30 and comparison with the analysis in line 15. In a second embodiment the total flow rate in line 15 is altered by changing the pumping rate from pump 24 to the optimum. By this means knowing the content of that component e.g. gas content on line 15, the optimum pump rate can be used.

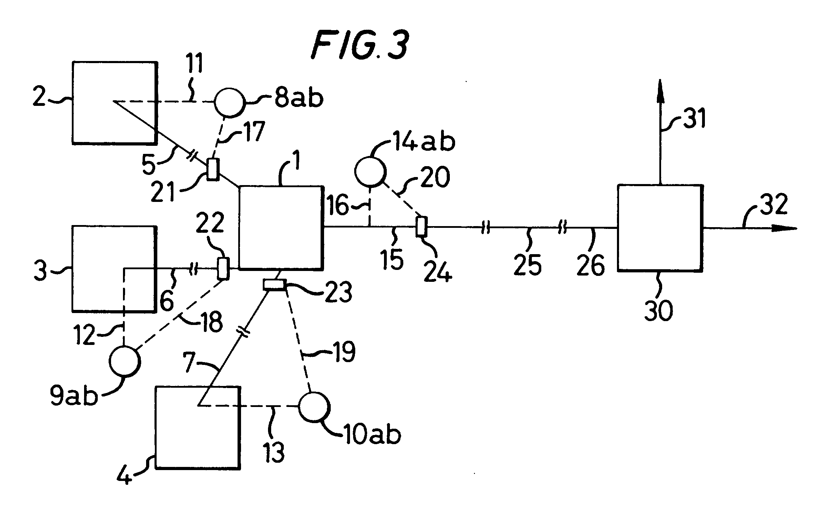

Fig.3 shows a combination of the processes of Figs. 1 and 2, in which the oils from locations 2, 3 and 4 pass via collection station 1, along pipelines 15 and 25 to separating means 30, from whence two fractions are removed in lines 31 and 32. The operations are as described for Figs. 1 and 2.

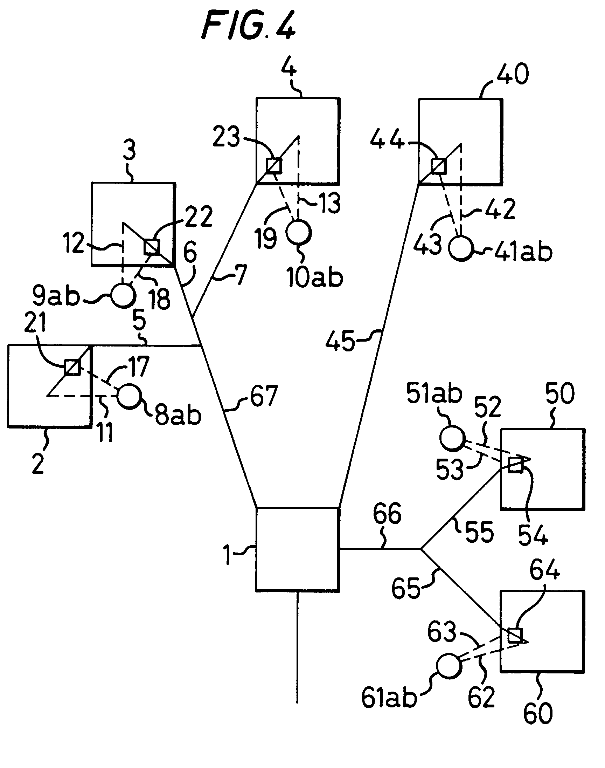

Referring now to Fig.4, there are three well head locations 2, 3 and 4 with equipment pipelines etc numbered as in Fig. 1 and also three more well head locations 40, 50 and 60, with pipelines 45, 55 and 65 towards collection station 1, analytical equipment 41ab, 51ab, 61ab (spectrometers and computers) joined to the pipelines 45, 55 and 65 respectively by analysis lines 42, 52 and 62 respectively, and signal transmission lines 43, 53 and 63 from computers 41b, 51b and 61b to pumps 44, 54 and 64 respectively. The general layout differs from that in Fig. 1 because of lines 5, 6 and 7 from well head locations 2, 3 and 4 meeting single line 67 which leads to collection station I and similarly lines 55 and 65 meeting single line 66 leading to collection station 1. The mode of operation of the apparatus in Fig.4 may be as for Fig. 1. If desired the blending process may be followed by the transport and separation process as in Fig.3.

However, in both Fig. 1 and Fig.4 apparatus the analysis of the oil at each well head may be used such (with the oil feed rate) in lines 5-7 etc to determine the amount of oil (and its components) from each well head reaching station 1 with or without control on that feed rate and hence any control on the composition of oil leaving the station 1.

The invention is illustrated in the following examples.

A crude oil containing dissolved gas was passed in a pipeline to a separator where its pressure was reduced and its components capable of being flashed off at 130°C under atmospheric pressure were separated. These components are gaseous hydrocarbons of 1-4 carbons, liquid gas condensate predominantly 5 carbon hydrocarbons and light ends, mostly 6 and 7 carbon alkanes. The crude oil was analysed before it entered the pipeline by a near infra red (NIR) spectrometer coupled to a computer to give absorbances that the computer converted to the content of the above components, so that the conditions of the separation i.e. temperature, pressure and flow rate could be controlled at the optimum for that separator with that crude. The conversion from absorbance to that content was performed using the correlation shown in the Regression Equation below. This equation had been determined earlier by calibration using a number of crude oils of known content of those components and their measured NIR absorbances and application of a multiple linear Regression (MLR) technique to correlate content of those components and NIR absorbances at specific wavelengths. The Regression Equation was as follows:

$$\text{Wt\% those components} = -[14505 \times \text{Abs1}]-[14534 \times \text{Abs2}]-[19857 \times \text{Abs3}]-[15370 \times \text{Abs4}]+[6577 \times \text{Abs5}]-[9128 \times \text{Abs6}]+466.7$$

where Abs1 etc is the normalised absorbance at the specific wavelength 1 etc.

The Tables below show the absorbances at particular wavelengths in relation to the content of those components (in weight %, based on the weight of the oil) for each of 4 crudes.

| Absorbance for Crude Oil x $10^{-3}$ | | | | | | |
|---|---|---|---|---|---|---|
| | Wavelength | | Brent | Forties | Kuwait | Jarn Yaphour |
| | $cm^{-1}$ | nm | | | | |
| 1 | 4600 | 2173 | 0.530 | 0.579 | 0.510 | 0.537 |
| 2 | 4356 | 2295 | 9.02 | 9.03 | 9.19 | 9.04 |
| 3 | 4228 | 2365 | 8.98 | 8.86 | 9.17 | 8.68 |
| 4 | 4156 | 2406 | 8.92 | 8.77 | 8.94 | 8.50 |
| 5 | 4100 | 2439 | 8.49 | 8.48 | 8.22 | 8.45 |
| 6 | 4000 | 2500 | 5.22 | 5.15 | 5.23 | 4.95 |

By application of the Regression Equation, the content of the above components was predicted, and compared to the values obtained by a standard technique.

| | % Above components (wt) | |
|---|---|---|
| Oil | Predicted | Found |
| Brent | 20.9 | 20.9 |
| Forties | 25.0 | 23.2 |
| Kuwait | 12.7 | 12.7 |
| Jarn Yaphour | 34.9 | 38.4 |

## Claims

1. A method of controlling a process for separating from a crude oil at least one component of said crude oil, which flows into a pipeline and then through a separator,

   Wherein a sample of the crude oil before entering the pipeline is analysed for at least one component on or at line to give an analytical result before the crude oil, from which the sample was taken, reaches the separator, and the operation of the separator is adjusted based on said result to control the separation of said component.

2. A method according to claim 1 wherein the sample of oil is taken and analysed at said pipeline at least 1Km from the separator.

3. A method according to claim 1 or 2 wherein the sample is analysed by chromatography or spectroscopy, preferably near infra red spectroscopy.

4. A method of determining or predicting a value of a property of a material which is a crude oil, or product of a process to which said crude oil is a feed or yield of said process, which comprises measuring the absorption of said oil at least one wavelength in the region 600-2600nm and converting that absorption (or derivative thereof) into a value of said property.

5. A method according to any one of the preceding claims wherein the crude oil contains dissolved gas.

6. A method according to claim 5 wherein the crude oil is analysed by near infra red spectroscopy and in real time the results are passed to the controller of a separator separating the dissolved gas from said oil in order to optimise operation of said separator.

7. A method according to any one of claims 1-4 wherein the crude oil contains naphtha, gas oil and fuel oil and the analysis determines the percentage of at least one thereof, preferably to determine the value of the crude oil.

8. A method according to any one of the preceding claims wherein the crude oil is analysed at or downstream of a well head.

9. A method according to claim 8 wherein crude oil flows from a series of well heads to a collecting station and NIR analysis are performed at each well head to determine the analysis for said component in the blend of oils made at the collection station.

10. A method according to anyone of the preceding claims where the crude oil sampled contains less than 10% wt insoluble solids and is analysed by transmission.

11. Apparatus for controlling a separation, which comprises a pipeline for transport of crude oil containing at least one component, separation means especially gas separation means, for separating said component, an analyser before at or on said pipeline for analysis of said crude oil for said component, a computer controlling said separation means and receiving signals from said analyzer for feedback control of said separation means.

FIG.1

FIG. 2

FIG.3

# FIG.4

| | European Patent Office | **EUROPEAN SEARCH REPORT** | Application Number |
|---|---|---|---|
| | | | EP 97 43 0007 |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,A | EP 0 706 041 A (BP CHEMICALS SNC) 10 April 1996<br>* page 2 *<br>--- | 1,3,4,6,11 | G01N33/28<br>G01N21/35 |
| A | US 5 266 800 A (MULLINS OLIVER C) 30 November 1993<br>* abstract *<br>--- | 3,4,6 | |
| A | GB 2 180 352 A (TEXACO DEVELOPMENT CORP) 25 March 1987<br>* page 1, line 21 - line 39 *<br>----- | 1,11 | |
| | | | TECHNICAL FIELDS SEARCHED (Int.Cl.6)<br><br>G01N<br>E21B |

The present search report has been drawn up for all claims

| Place of search | Date of completion of the search | Examiner |
|---|---|---|
| THE HAGUE | 11 July 1997 | Scheu, M |

EPO FORM 1503 03.82 (P04C01)